# EUROPEAN PATENT APPLICATION

(11) **EP 1 767 161 A1**
(43) Date of publication of application: **28.03.2007**
(21) Application number: 06254851.6
(22) Date of filing: 19.09.2006
(51) Int. Cl.: A61B 17/88, A61B 17/70

(54) **Spinal fixation rod contouring system**

(30) Priority: 22.09.2005 US 719380 P
(71) Applicant: Zimmer Spine, Inc., Minneapolis, MN 55439-2027 (US)
(72) Inventor: Hestad, Hugh D., Edina MN 55410 (US); Emstad, Erik E., St. Paul, MN 55104 (US)
(74) Representative: Findlay, Alice Rosemary

(57) **Abstract**

A method and associated system of implanting a vertebral anchor such as a pedicle screw assembly (10a-c) includes the steps of implanting a number of pedicle screws (10a-c) into a series of vertebrae (V1-V3); attaching a number of provisional posts (14a-c) to respective pedicle screws, each provisional post including a provisional saddle (12a-c); bending a rod (20) to conform to a desired alignment of the provisional saddles (12a-c); removing the provisional posts from the pedicle screw heads (11a-c); and connecting the bent rod to the pedicle screw heads. Advantageously, the method allows external fitting and sizing of a fixation rod before it is implanted and secured to the vertebral anchors via a minimally invasive manner.

## Description

This invention relates to a system for vertebral stabilization using vertebral anchors and fixation rods.

Conventional vertebral stabilization surgery using vertebral anchors and fixation rods requires a surgeon to prepare a long incision aligned with the vertebral column of a patient. Pedicle screws are then inserted into a number of vertebrae after which a fixation rod is located with respect to saddles attached to the pedicle screws. The fixation rod is then bent to match the orientation of the pedicle screw heads and/or the desired curvature of the spine. Visualization of the accuracy of the alignment of the rod and the screw heads may be difficult because of visual interference from tissue and blood, for example. Conventional surgical methods require a large midline incision and retraction of skin and muscle to provide the surgeon with sufficient visualization of the pedicle bone structure.

Improved methods, systems and devices that address these and other shortcomings in the prior art are needed.

The systems, devices and methods described herein are used to aid in surgery for vertebral stabilization using vertebral anchors and fixation rods. Currently, this type of surgery is often performed using pedicle screws that have top loading saddles to receive rods wherein the rods are bent to match the orientation of the pedicle screw heads and/or the desired curvature of the spine. Rod bending is performed once the screws are placed into vertebrae and, therefore, visualization of the accuracy of the alignment of the rod with the screw heads is difficult. A surgical method using the system of this invention would follow the same course as traditional surgery except the tissue would not need to be cut between the pedicle screw heads prior to the rods being sized and bent.

According to embodiments of this invention, provisional pedicle screw posts are attached to pedicle screw heads that would either be cannulated and have an attachment mechanism, or simply have an interlocking fit, to receive the provisional pedicle screw posts with the pedicle screw head saddle. The provisional pedicle screw posts may have links that connect each post to an adjacent post such that the adjacent posts are parallel to each other. The links and posts may be keyed or have other means to maintain the posts in parallel arrangement with each other. Once the posts are linked together, a mock, provisional, or trial saddle is placed on the proximal end of each provisional post. These mock saddles may have an indexed feature to ensure placement in the same trajectory as the pedicle screw saddles. A straight rod may then be placed on the outermost provisional posts. Based on the distance between the center saddle and the rod, the surgeon can determine how much the rod needs to be bent. The rod can then be bent and placed into the provisional saddles to check alignment as well as cut the rod to a proper length.

Once the modification of the rod is complete, i.e., bending and cutting to length, the tissue can be cut between the pedicle screws and dissected in a minimally invasive manner for placement of the rod followed by closing the incision after rod placement and securing. The rod may come in a series of varying radiuses, i.e., pre-bent, so the surgeon could simply place a pre-bent rod into the provisional saddles and change the rod with a different pre-bent rod as needed. The amount of pre-bending of the rod may be undersized with respect to the saddle trajectory to create more lordosis on the construct when the rod is fixed in place.

One advantage of the system of this invention is that the pedicle screws could be placed, and the rod modified, sized and bent using this device, after which a minimally invasive interconnecting incision is made to the pedicle screw heads so the rod can be placed and tightened. Alternatively, the modified rod may be inserted into the first incision and fed through all of the pedicle screw head saddles. Either of these insertion methods may potentially reduce the surgical time, as well as reduce necessity of significant tissue dissection and retraction during surgery.

Another advantage of this system is the increased visualization of the fit of the rod to the pedicle screw head saddles. Very often, this is instrumental in achieving the intended outcome. In traditional surgery, visualization of the actual fit of these components is difficult because of blood and tissue. In addition, elimination of the need to significantly retract tissue is a large benefit that could provide a quicker recovery time.

Additional features may include an apparatus for determining the trajectory of the connecting rod by use of a modified compass mechanism for extrapolating the correct radius and any obtuse angles that may be encountered with various anatomies. Again, the rods may be checked for correct alignment and length in the mock saddles.

There is disclosed a method of implanting a pedicle screw assembly including the steps of implanting a plurality of pedicle screws into a series of vertebrae; attaching a plurality of provisional posts to respective pedicle screws, each provisional post including a provisional saddle; bending a rod to conform to a desired alignment of the provisional saddles; removing the provisional posts from the pedicle screw heads; and connecting the bent rod to the pedicle screw heads.

The invention will now be further described by way of example with reference to the accompanying drawings in which:

Fig. 1 is a view of a portion of a spinal column including a plurality of vertebrae, and further showing various components of the pedicle fixation rod alignment system of the present invention;

Fig. 2 is a perspective view of a provisional or pedicle screw head saddle;

Fig. 3 is a cross-sectional view of a portion of Fig. 1, further illustrating the connection between the provisional post, the pedicle screw, and the pedicle screw head saddle; and

Fig. 4 is a cross-sectional view of a portion of Fig. 1, further illustrating a connection between a provisional saddle and a provisional post.

Corresponding reference characters indicate corresponding parts throughout the several views. The exemplification set out herein illustrates embodiments of the invention and such exemplification is not to be construed as limiting.

In general, the system of this invention provides a trial assembly for trialing a pedicle fixation rod exteriorly of a patient during surgery.

The present method and devices employ similar minimally invasive surgical methods and devices as described in U.S. Patent Application Serial No. 11/228,958, entitled APPARATUS AND METHOD FOR MINIMALLY INVASIVE SPINE SURGERY, filed on September 16, 2005.

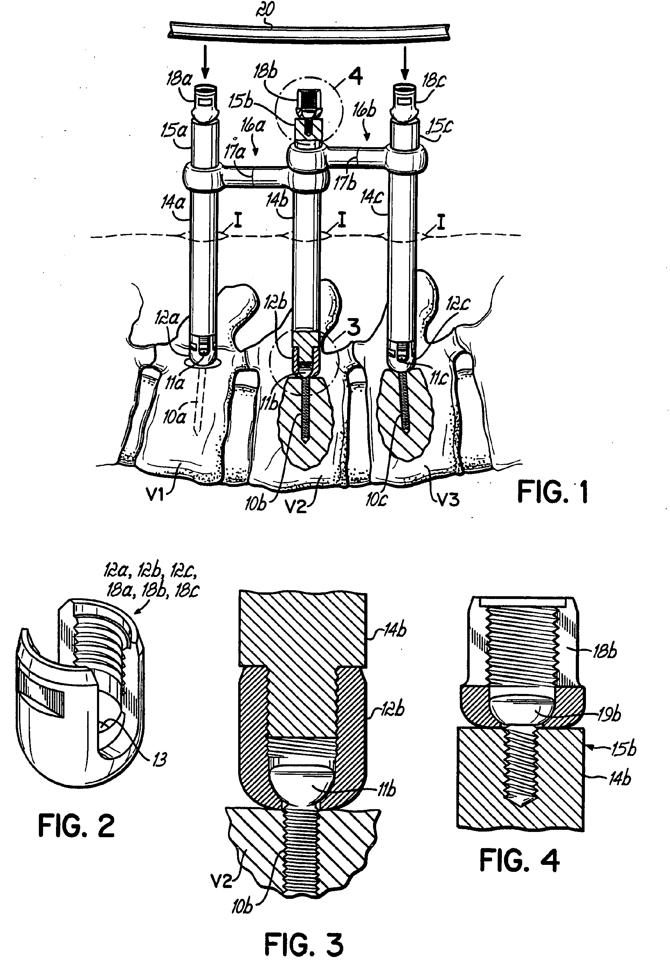

Referring now to Fig. 1, a spinal fixation construct according to various embodiments of this invention includes a number of vertebral anchors in the form of pedicle screws 10a, 10b, 10c having pedicle screw heads 11a, 11b, 11c, respectively, positioned in vertebrae V1, V2, and V3 after a surgeon makes a plurality of small incisions I in the skin of the patient proximate vertebrae V1, V2, and V3. The pedicle screws 10a, iob, 10c include pedicle screw head saddles 12a, 12b, 12c, respectively, shown in Figs. 1-2, each of which typically includes an aperture 13 (Fig. 2) in the bottom thereof through which each pedicle screw 10a, 10b, 10c passes. Provisional, trial, or mock pedicle screw posts 14a, 14b, 14c are placed onto the pedicle screw heads 11a, 11b, 11c, respectively, via engagement with the pedicle screw head saddles 12a, 12b, 12c, respectively. The engagement of the provisional posts 14a, 14b, 14c with respective saddles 12a, 12b, 12c may be any attachment mechanism, such as a threading engagement shown in Fig. 3, a snap-fit engagement, or an interlocking fit engagement, for example. The provisional posts 14a, 14b, 14c may be cannulated.

The provisional pedicle screw posts 14a, 14b, 14c extend externally of the patient or percutaneously and are linked together via an alignment arrangement which in one embodiment includes links 16a, 16b, wherein link 16a connects provisional post 14a to an adjacent provisional post 14b such that adjacent posts 14a and 14b are parallel to each other. Similarly, link 16b connects provisional post 14b to an adjacent provisional post 14c such that adjacent posts 14b and 14c are parallel to each other. This is accomplished by having the links 16a, 16b and the provisional posts 14a, 14b, 14c keyed or include any other mechanism to maintain the provisional posts 14a, 14b, 14c parallel to each other. The links 16a and 16b must be radially lockable with respect to the posts 14a, 14b, 14c. The length of each link 16a, 16b may be adjustable via telescoping joints 17a and 17b which must also be lockable.

Once the provisional posts 14a, 14b, 14c are linked together, provisional, trial, or mock saddles 18a, 18b, 18c (Figs. 1-2) are placed on proximal ends 15a, 15b, 15c of each respective provisional post 14a, 14b, 14c. The connection of the provisional saddles 18a, 18b, 18c with respective the provisional posts 14a, 14b, 14c may be via any suitable connecting arrangement, such as a connecting screw 19b shown in Fig. 4, a thread-type engagement, or a snap-fit engagement. Alternatively, the provisional saddles 18a, 18b, 18c may be integrally formed with the provisional posts 14a, 14b, 14c and made of stainless steel. The provisional saddles 18a, 18b, 18c may have an indexed feature to place them in the same trajectory as the pedicle screw head saddles 12a, 12b, 12c. Additionally, the provisional saddles 18a, 18b, 18c have identical geometries as respective pedicle screw head saddles 12a, 12b, 12c. Next, a rod 20 can be placed in each of the provisional saddles 18a, 18b and 18c or only on the provisional saddles 18a, 18c of the outermost provisional posts, i.e., provisional posts 14a and 14c. Based on the distance between the provisional saddle 18b on the provisional post 14b and the rod 20, the surgeon determines how much the rod 20 needs to be bent. The rod 20 can then be bent and placed into the provisional saddles 18a, 18b, and 18c to check alignment as well as to be cut for proper length.

Advantageously, the system facilitates replication of the exact relative geometry and positioning of the pedicle screw head saddles 12a, 12b, 12c attached to the respective vertebrae by using the provisional pedicle screw posts 14a, 14b, 14c, the provisional saddles 18a, 18b, 18c, and the links 16a, 16b. The external replication of the internal geometry and positioning of the saddles 12a, 12b, and 12c allows a surgeon to use minimally invasive surgical techniques to implant the rod 20, thereby reducing the necessity for significant tissue dissection and retraction.

Once the sizing of the rod 20 is complete, the tissue can be cut between the pedicle screws 10a, 10b, 10c in a minimally invasive manner with minimal or no tissue retraction so that the rod 20 can be placed and tightened onto each pedicle screw 10a, 10b, 10c via appropriate set screws (not shown) followed by closing of the incision. The rod 20 may come in a series of varying radiuses, i.e., pre-bent, so that the surgeon could simply place the pre-bent rods into the provisional saddles 18a, 18b, 18c and change size as needed. This could also be used to undersize the radius of the rod 20 versus the saddle trajectory to create more lordosis on the construct when the rod 20 is placed and tightened.

One advantage of this system and device is that the pedicle screws 10a, 10b, 10c may be placed into the vertebrae V1, V2, and V3, and the rod 20 is sized and bent using this device, after which a minimally invasive interconnecting incision is made to the pedicle screw heads 11a, 11b, 11c so that the rod 20 can be placed and tightened. This could potentially reduce the surgical time, as well as reduce the necessity of significant tissue dissection and retraction during surgery. Alternatively, after the rod 20 is bent to the correct size, the rod 20 may be inserted through the first incision, i.e., the incision I made for insertion of pedicle screw 10a, and fed through the pedicle screw head saddles 12a, 12b, 12c without requiring any additional incision of tissue, thereby advantageously reducing the amount of trauma inflicted on a patient during surgery.

Another advantage of this system is the increased visualization of the fit of the rod 20 to the pedicle saddles 12a, 12b, 12c which is instrumental in achieving the intended outcome. In traditional surgery, it is typically difficult to see the actual fit of these components because of blood and tissue. In addition, the elimination of the need to significantly retract tissue is a large benefit that could provide a quicker recovery time.

In an alternative embodiment, the system could include an apparatus (not shown) for determining the trajectory of the connecting rod 20 by use of a modified compass mechanism that could be connected to the provisional pedicle screw posts 14a, 14b, 14c and extrapolate the correct radius as well as obtuse angles that may be encountered with various anatomies. Then, these values could be used to properly bend the rod 20. Again, the rod 20 could be checked in the provisional saddles 18a, 18b, 18c prior to making the interconnecting incision.

Although the invention is described throughout as being applied to three vertebrae, i.e., a two-level system, the invention may be used with any number of vertebrae that need to be fixed together by a fixation system. Additionally, this invention may also be used in transverse fixation systems. Additionally, the pedicle screws are shown and described herein, but other types of vertebral anchors could be utilized such as hooks and other vertebral anchor mechanisms.

## Claims

1. A system for installing a spinal fixation construct, the system comprising:
a plurality of vertebral anchors each adapted to be securely coupled to a selected vertebrae of a patient;
a plurality of provisional posts each selectively coupled to one of the vertebral anchors and adapted to extend percutaneously from the patient when coupled to the associated vertebral anchor;
a plurality of provisional saddles each coupled to one of the provisional posts and adapted to be positioned super-cutaneously when coupled to the associated provisional post; and
a rod adapted to be modified according to the position of at least some of the provisional saddles coupled to the associated provisional posts coupled to the vertebral anchors coupled to the vertebrae of the patient;
wherein the provisional posts and provisional saddles are adapted to be removed from the associated vertebral anchors and the patient after the rod is modified;
wherein the modified rod is adapted to be secured to the vertebral anchors coupled to the associated vertebrae after the provisional posts are removed from the associated vertebral anchors.

2. The system of claim 1 wherein the vertebral anchors are pedicle screws and each pedicle screw further comprises:
a saddle adapted to receive and secure the modified rod.

3. The system of either claim or claim 2 further comprising:
an alignment arrangement adapted to be positioned super-cutaneously and coupled to selected adjacent provisional posts to maintain a relative position of the adjacent provisional posts.

4. The system of claim 3 wherein the alignment arrangement further comprises:
a link adapted to be coupled to each of the adjacent posts.

5. The system of claim 4 wherein a length of the link is adjustable.

6. The system of any preceding claim further comprising:
a threaded engagement between each provisional post and the associated vertebral anchor.

7. The system of any preceding claim wherein each provisional saddle is selectively coupled to the associated provisional post.
